# EUROPEAN PATENT SPECIFICATION

(11) **EP 3 357 436 B1**
(45) Date of publication and mention of the grant of the patent: **30.12.2020**
(21) Application number: 18155158.1
(22) Date of filing: 05.02.2018
(51) Int. Cl.: A61B 17/128

(54) **ENDOSCOPIC SURGICAL CLIP APPLIER**
ENDOSKOPISCHER CHIRURGISCHER KLAMMERAPPLIKATOR
APPLICATEUR ENDOSCOPIQUE D'AGRAFES CHIRURGICALES

(30) Priority: 06.02.2017 US 201762455090 P; 09.01.2018 US 201815865843
(43) Date of publication of application: 08.08.2018
(73) Proprietor: Covidien LP, Mansfield, MA 02048 (US)
(72) Inventor: WILLIAMS, Justin, Southbury, Connecticut 06488 (US)
(74) Representative: Maschio, Antonio

(56) References cited:
- EP-A2- 2 752 165
- WO-A1-2013/040378
- WO-A1-2016/192096
- US-A- 5 755 726
- US-A- 6 053 908
- US-A1- 2006 079 913
- US-A1- 2007 049 949

## Description

### CROSS-REFERENCE TO RELATED APPLICATION

This application claims the benefit of and priority to U.S. Provisional Patent Application No. 62/455,090 filed February 6, 201 7.

### BACKGROUND

### Technical Field

The present disclosure relates generally to surgical clip appliers. More particularly, the present disclosure relates to endoscopic surgical clip appliers having a release switch for a ratchet assembly thereof.

### Description of Related Art

Endoscopic surgical staplers and surgical clip appliers are used for a number of minimally invasive or endoscopic surgical procedures. Typically in a minimally invasive surgical procedure, a tube or cannula device is extended into the patient's body through the entrance incision to provide an access port. The port allows the surgeon to insert a number of different surgical instruments therethrough for performing surgical procedures far removed from the incision.

Endoscopic surgical clip appliers are capable of applying a single or multiple surgical clips during a minimally invasive surgical procedure. Applying surgical clips usually involves compressing the clip over a vessel. Once applied to the vessel, the compressed surgical clip terminates the flow of fluid therethrough. Terminating the flow of fluid through a vessel typically requires complete formation of the surgical clip.

During certain endoscopic procedures, it may be desirable and/or necessary to partially form clips. For example, a partially formed clip may be used to secure a catheter around tissue during a cholangiogram or other medical procedure.

Accordingly, a need exists for endoscopic surgical clip appliers that provide a clinician with a convenient way to partially form surgical clips.

US 2007049949 (A1) discloses an advancement block for a clip applying device. The advancement block includes a body that proximally slides in the axial direction when a trigger is squeezed, and further distally slides in the axial direction when the trigger is released, and a toggle pivotably connected to the body, the toggle pivoting in a first direction about a pivot axis generally orthogonal to the axial direction and to a first position when the body proximally slides in the axial direction, the toggle further pivoting in a second direction about the pivot axis and to a second position when the body distally slides in the axial direction. When in the second position, the toggle engages and distally moves the ladder member such that the ladder member distally moves the rearmost clip in the axial direction.

WO2013040378 (A1) discloses an applier for ligation clip. The applier includes: an outer tube having mounting bosses; a pair of jaws pivotally connected to the mounting bosses, the jaws having actuating projections, a feed tube located in the outer tube and configured to move axially within the outer tube, the feed tube having actuating slots in which the actuating projections are located; a clip lock arm located in the outer tube and configured to move axially within the outer tube; and a clip advance arm located in the outer tube and configured to move axially within the outer tube, the clip advance arm having flexible pinchers at one end of the clip advance arm. A method of applying a ligation clip is also disclosed.

### SUMMARY

The present disclosure relates to endoscopic surgical clip appliers that allow a clinician to choose whether to partially or completely form surgical clips.

According to the invention, an endoscopic surgical clip applier includes an endoscopic assembly and a handle assembly. The endoscopic assembly includes a shaft assembly and a pair of jaw members operatively coupled to, and extending from the shaft assembly. The handle assembly includes a housing selectively connectable to the endoscopic assembly. A fixed handle extends from the housing, and a trigger is pivotally connected to the fixed handle. A drive bar is disposed within the housing of the handle assembly and is operatively coupled to the trigger and to the pair of jaw members to move the pair of jaw members between a spaced apart configuration and an approximated configuration upon actuation of the trigger. A ratchet assembly is also disposed within the housing of the handle assembly. The ratchet assembly includes a first rack operatively coupled to the drive bar. The first rack defines a plurality of first rack teeth, has a distal end and a proximal end, and includes a first length between the distal end and the proximal end thereof. A second rack is operatively coupled to the drive bar, spaced apart from the first rack. The second rack defines a plurality of second rack teeth, has a distal end and a proximal end, and includes a second length between the distal end and the proximal end thereof. The second length of the second rack is less than the first length of the first rack.

The ratchet assembly further includes a first pawl mounted within the housing of the handle assembly, the first pawl being selectively engagable with the plurality of first rack teeth of the first rack in a first position thereof.

Further, the ratchet assembly further includes a second pawl mounted within the housing of the handle assembly, the second pawl being selectively engageable with the plurality of second rack teeth of the second rack in a first position thereof.

In embodiments, the ratchet assembly further includes a distal well disposed adjacent the distal end of the first rack, wherein the first pawl is located in the distal well in an un-actuated position of the trigger.

In some embodiments, the ratchet assembly further includes a proximal well disposed between the proximal end of the first rack and the distal end of the second rack, wherein the second pawl is located in the proximal well in the un-actuated position of the trigger.

In embodiments, the first rack is disposed in a position distal of the second rack.

According to the invention, the endoscopic clip applier further includes a release switch at least partially supported within the housing of the handle assembly and operatively associated with the first pawl. The release switch is selectively actuatable to move the first pawl from the first position, wherein the first pawl is in registration with the plurality of first rack teeth of the first rack, to a second position, wherein the first pawl is out of registration with the plurality of first rack teeth of the first rack.

In embodiments, when the release switch is actuated, the second pawl maintains registration with the plurality of second rack teeth of the second rack, in the first position thereof, until the second pawl is disposed in the proximal well or until the second pawl is disposed proximally beyond the proximal end of the second rack.

In some embodiments, the drive bar is longitudinally movable upon actuation of the trigger, wherein as the drive bar is moved longitudinally in a first direction, and the release switch is not actuated, the first pawl and the second pawl are moved over the plurality of first rack teeth and the plurality of second rack teeth of the first and the second racks, respectively, such that longitudinal movement of the drive bar in a second, opposite, direction is prevented until the first pawl is disposed in the distal well and the second pawl is disposed in the proximal well or until the first pawl is disposed at the proximal end of the first rack and the second pawl is disposed proximally beyond the proximal end of the second rack.

In embodiments, the drive bar is longitudinally movable upon actuation of the trigger, wherein as the drive bar is moved longitudinally in a first direction, and the release switch is actuated to move the first pawl out of registration with the plurality of first rack teeth of the first rack, longitudinal movement of the drive bar in a second, opposite, direction is prevented until the second pawl is disposed in the proximal well or until the second pawl is disposed proximally beyond the proximal end of the second rack.

In embodiments, as the drive bar is moved longitudinally in the first direction, and the release switch is actuated to move the first pawl out of registration with the plurality of first rack teeth of the first rack, the second pawl is disposed beyond the proximal end of the second rack as the trigger reaches a partially actuated position, wherein the drive bar is longitudinally movable in the second, opposite, direction, as the trigger reaches a fully un-actuated position from the partially actuated position.

In embodiments, the endoscopic assembly further includes a plurality of surgical clips slidably disposed within the shaft assembly and selectively formable between the pair of jaw members, wherein when the first pawl is in the first position, the first pawl is in registration with the first rack disposed on the drive bar such that upon actuation of the trigger, the trigger is prevented from reversing the direction of movement thereof until the trigger is moved to a fully actuated position and a distal most surgical clip of the plurality of surgical clips is fully formed between the pair of jaw members.

In some embodiments, when the first pawl housing is in the second position, the first pawl is out of registration with the first rack disposed on the drive bar such that when the second pawl is disposed beyond the proximal end of the second rack and the trigger is moved to the partially actuated position, the trigger is capable of reversing the direction of movement thereof such that the distal most surgical clip of the plurality of surgical clips is partially formed between the pair of jaw members.

In embodiments, the ratchet assembly further includes a first pawl spring and a second pawl spring supported within the housing of the handle assembly. The first pawl spring is configured to bias the first pawl into engagement with the plurality of first rack teeth of the first rack, and the second pawl spring being is to bias the second pawl into engagement with the plurality of second rack teeth of the second rack.

In embodiments, the first pawl includes a lockout member extending therefrom. The lockout member selectively engages the release switch as the trigger moves to the fully un-actuated position, to prevent the release switch from moving the first pawl out of registration with the plurality of first rack teeth of the first rack, to the second position thereof.

In embodiments, the ratchet assembly further includes a release spring supported in the handle assembly. The release spring is operatively associated with the release switch and is biased to return the release switch to a home position such that the release switch is disengaged from the first pawl.

According to another aspect of the present disclosure, an endoscopic surgical clip applier includes an endoscopic assembly and a handle assembly. The endoscopic assembly includes a shaft assembly and a pair of jaw members operatively coupled to, and extending from the shaft assembly. The handle assembly includes a housing selectively connectable to the endoscopic assembly, a fixed handle extending from the housing, and a trigger pivotally connected to the fixed handle. A drive bar is disposed within the housing of the handle assembly and is operatively coupled to the trigger and the pair of jaw members to move the pair of jaw members between a spaced apart configuration and an approximated configuration upon actuation of the trigger. A ratchet assembly is also disposed within the housing of the handle assembly. In embodiments, the first rack is defined on a top portion of the drive bar, the second rack is defined on the top portion of the drive bar. Upon movement of the trigger, a reversal of a direction of movement of the trigger is prohibited until the second pawl is disposed distally beyond the distal end of the second rack or proximally beyond the proximal end of the second rack.

### BRIEF DESCRIPTION OF THE DRAWINGS

Particular embodiments of endoscopic surgical clip appliers are described herein with reference to the drawings wherein:
FIG. 1 is a perspective view of an endoscopic surgical clip applier, according to the present disclosure including an endoscopic assembly and a handle assembly;
FIG. 2A is a side view, of the handle assembly of FIG. 1 with at least a housing half-section removed therefrom;
FIG. 2B is a perspective view, with parts separated, of the handle assembly of FIG. 1;
FIG. 3A is a side view, of the handle assembly of FIG. 2A, illustrating a ratchet assembly thereof, in an initial/reset position of a normal actuation of the ratchet assembly;
FIG. 3B is a side view, of the handle assembly of FIG. 2A, illustrating the ratchet assembly of FIG. 3A, in a first configuration of the normal actuation of the ratchet assembly;
FIG. 3C is a side view, of the handle assembly of FIG. 2A, illustrating the ratchet assembly of FIG. 3A, in a second configuration of the normal actuation of the ratchet assembly;
FIG. 3D is a side view, of the handle assembly of FIG. 2A, illustrating the ratchet assembly of FIG. 3A, in a third configuration of the normal actuation of the ratchet assembly;
FIG. 4A is a side view, of the handle assembly of FIG. 2A, illustrating the ratchet assembly thereof, in a first configuration of a partial actuation of the ratchet assembly;
FIG. 4B is a side view, of the handle assembly of FIG. 2A, illustrating the ratchet assembly of FIG. 4A, in a second configuration of the partial actuation of the ratchet assembly;
FIG. 4C is a side view, of the handle assembly of FIG. 2A, illustrating the ratchet assembly of FIG. 4A, in a third configuration of the partial actuation of the ratchet assembly;
FIG. 5A is a side view, of the handle assembly of FIG. 2A, illustrating the ratchet assembly thereof, in a first configuration of a return stroke of the normal actuation of ratchet assembly;
FIG. 5B is a side view, of the handle assembly of FIG. 2A, illustrating the ratchet assembly of FIG. 5A, in a second configuration of the return stroke of the normal actuation of ratchet assembly;
FIG. 5C is a side view, of the handle assembly of FIG. 2A, illustrating the ratchet assembly of FIG. 5A, in a third configuration of the return stroke of the normal actuation of ratchet assembly; and
FIG. 6 is a perspective view of the endoscopic assembly of FIG. 1, with parts separated.

### DETAILED DESCRIPTION OF EMBODIMENTS

In accordance with the present disclosure, an endoscopic surgical clip applier includes a ratchet assembly having a first rack, with a first length, operatively associated with a first pawl, a second rack, with a second length less than the first length of the first rack, operatively associated with a second pawl, and a release switch operatively associated with the first pawl. In embodiments, upon actuation of a trigger, the first and second pawls are configured to engage a plurality of first and second rack teeth of the first and second racks, respectively, to prohibit release and reversal of a direction of movement of the trigger until the first and second pawls are disposed within respective clearances of the first and second racks. In embodiments, the release switch is selectively actuatable to move the first pawl out of registration or engagement with the plurality of first rack teeth of the first rack such that the direction of movement of the trigger may be reversed early once the second pawl has traversed the second, lesser length of the second rack. It is contemplated that the release switch may be useful to partially form clips, if desired for example, to secure a catheter around tissue during a cholangiogram or other medical procedure.

Embodiments of endoscopic surgical clip appliers, in accordance with the present disclosure, will now be described in detail with reference to the drawing figures wherein like reference numerals identify similar or identical structural elements. As shown in the drawings and described throughout the following description, as is traditional when referring to relative positioning on a surgical instrument, the term "proximal" refers to the end of the apparatus which is closer to the user and the term "distal" refers to the end of the apparatus which is further away from the user.

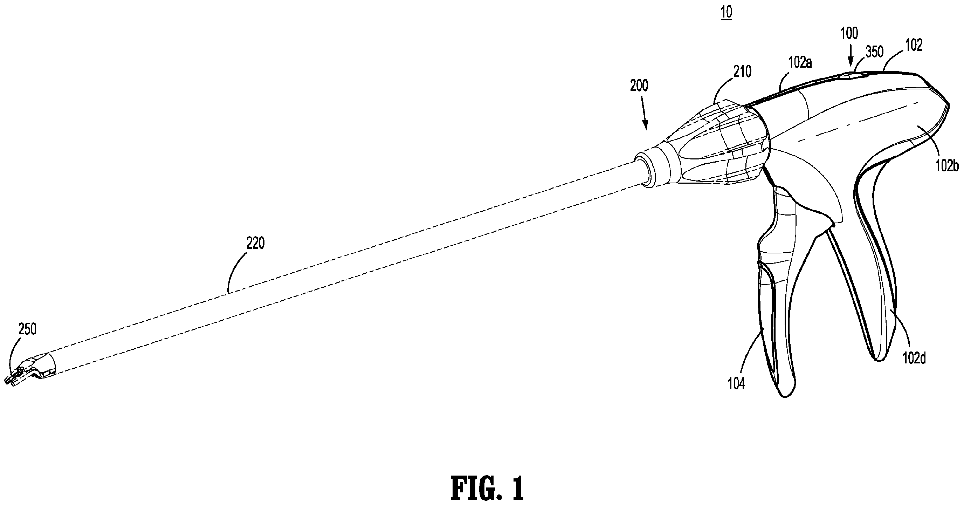

Referring now to FIGS. 1-6, an endoscopic surgical clip applier in accordance with an embodiment of the present disclosure is generally designated as 10. Surgical clip applier 10 generally includes a handle assembly 100 and an endoscopic assembly 200 that extends distally from handle assembly 100. Generally, endoscopic assembly 200 includes a hub assembly 210, a shaft assembly 220 extending from hub assembly 210, and a pair of jaws 250 pivotally connected to a distal end of shaft assembly 220. Optionally, at least one disposable surgical clip cartridge assembly (not shown) may be selectively loadable into shaft assembly 220 of endoscopic assembly 200.

Referring now to FIGS. 1-2B, handle assembly 100 includes a housing 102 having a first or right side half-section 102a and a second or left side half-section 102b. Housing 102 of handle assembly 100 further includes or defines a nose 102c for supporting hub assembly 210 of endoscopic assembly 200, and a fixed handle 102d.

Housing 102 of handle assembly 100 may be formed of a suitable polymer, plastic or thermoplastic material. It is further contemplated that housing 102 of handle assembly 100 may be fabricated from stainless steel or the like.

Handle assembly 100 includes a trigger 104 pivotably supported between right side half-section 102a and left side half-section 102b of housing 102. Trigger 104 is pivotably movable in a first direction such that the trigger 104 and the fixed handle 102d are approximated and pivotably movable in a second, opposite, direction such that the trigger 104 and the fixed handle 102d are spaced-apart.

A drive bar 106 is supported within the housing 102 of the handle assembly 100. The drive bar 106 is a substantially flat member having a distal end portion 106a and a proximal end portion 106b. The distal end portion 106a of drive bar 106 includes a hook member 114 that is provided to mate with a feature of endoscopic assembly 200. The drive bar 106 is operatively coupled to the trigger 104 and the pair of jaws 250 of endoscopic assembly 200 to move the pair of jaws 250 between a spaced-apart configuration and an approximated configuration upon actuation of the trigger 104. Specifically, the handle assembly 100 includes a wishbone link 108 configured to couple the trigger 104 and the drive bar 106. Wishbone link 108 includes a first end portion having a tail 108a and a second end portion having a first arm and a second arm 108b, 108c spaced-apart to define a space 108d therebetween. The tail 108a of the wishbone link 108 is pivotably connected to trigger 104 through a trigger slot 104a. Specifically, tail 108a of wishbone link 108 includes an opening 108f configured for pivotably locating a pin (not specifically shown) defined within trigger slot 104a. The space 108d between the first and second arms 108b, 108c is configured to receive the drive bar 106. The first and second arms 108b, 108c of wishbone link 108, and the drive bar 106 includes corresponding apertures 108e, 106c, respectively, configured to locate a drive bar pin 110 to pivotably connect the wishbone link 108 and the drive bar 106. The wishbone link 108 is configured to translate the pivotal movement of the trigger 104 into longitudinal movement of the drive bar 106, as will be detailed below.

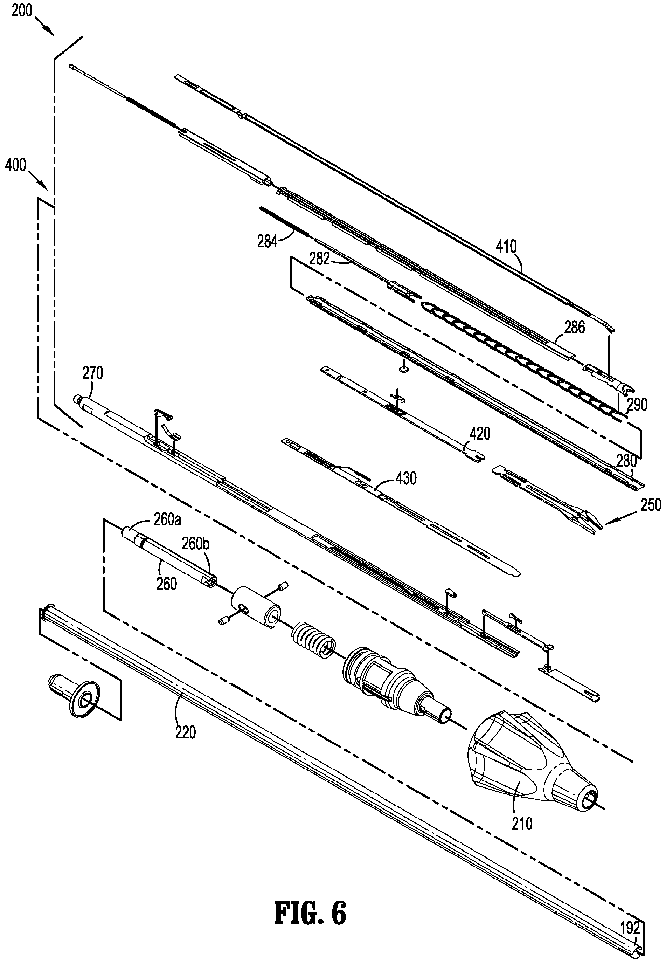

The drive bar 106 is configured to move one or more driving structures to load, and actuate the pair of jaws 250 to form a clip 290 (see FIG. 6) fully or partially, and then reset to an initial position for the next clip application. To achieve this, a biasing member, such as, for example, a first return spring 112 is disposed to surround the drive bar 106 adjacent the distal end portion 106a such that, after the trigger 104 is actuated and the wishbone link 108 advances the drive bar 106 in a longitudinal or distal manner, the first return spring 112 is provided to return the drive bar 106 and the trigger 104 to its original position for the next clip application. Further, in one embodiment, a second return spring 113 is disposed in housing 102 of handle assembly 100 and configured to operatively connect the trigger 104 and the fixed handle 102d such that, after the trigger 104 is actuated, the second return spring 113 is provided to return the trigger 104 to its original position.

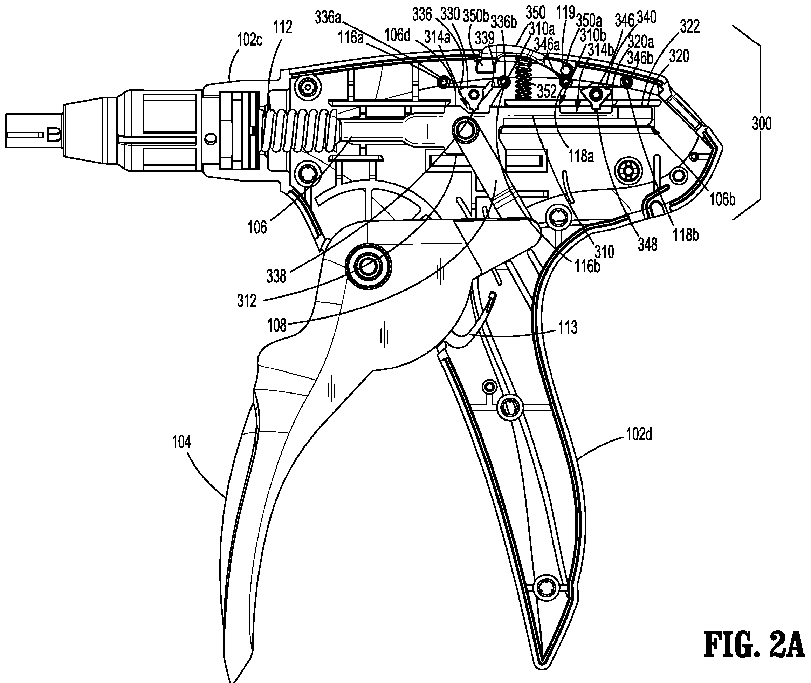

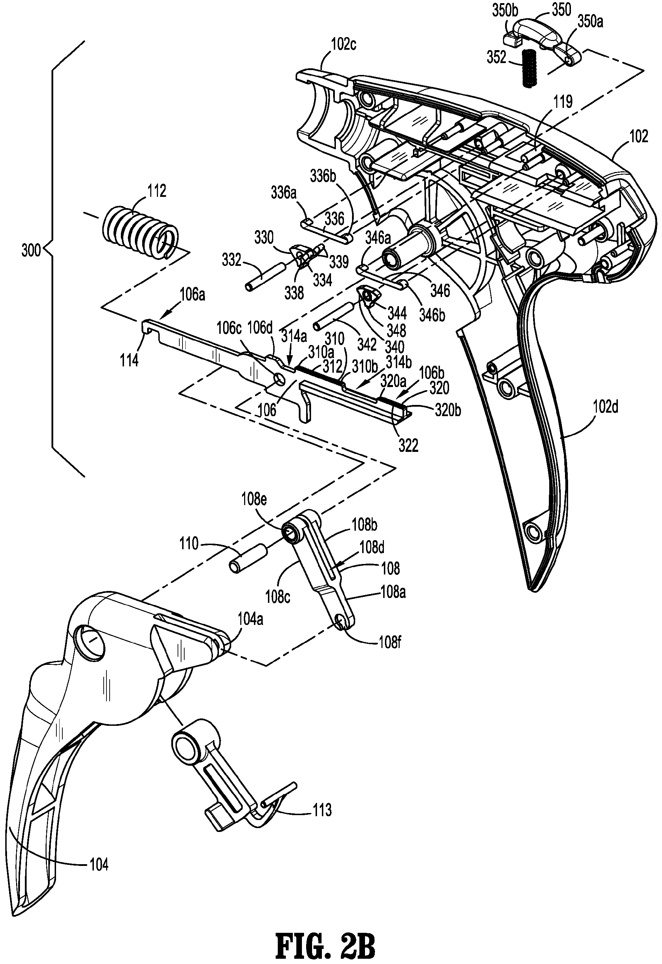

With continued reference to FIGS. 2A and 2B, surgical clip applier 10 includes a ratchet assembly 300 disposed within housing 102 of handle assembly 100. The ratchet assembly 300 generally includes a first rack 310, a second rack 320, a first pawl 330, a second pawl 340, and a release switch 350 at least partially supported within housing 102 of handle assembly 100.

As shown in FIGS. 2A and 2B, the first and second racks 310, 320 are supported by or are provided on a top surface 106d of the drive bar 106. The first rack 310 includes a distal end 310a and a proximal end 310b. The first rack 310 defines a plurality of first rack teeth 312 in series between the distal end 310a and the proximal end 310b thereof. Similarly, the second rack 320 includes a distal end 320a and a proximal end 320b. The second rack 320 defines a plurality of second rack teeth 322 in series between the distal end 320a and the proximal end 320b thereof.

The top surface 106d of the drive bar 106 also includes a distal clearance or well 314a located adjacent the distal end 310a of the first rack 310 and a proximal clearance or well 314b located between the proximal end 310b of the first rack 310 and the distal end 320a of the second rack 320. The distal well 314a is configured to receive the first pawl 330 and the proximal well 314b is configured to receive the second pawl 340 in an initial and/or reset position, as will be detailed further.

As shown in FIG. 2B, the first and second pawls 330, 340 are pivotably mounted within the handle assembly 100 between the right side half-section 102a and the left side half-section 102b of the housing 102. Specifically, the first pawl 330 is pivotably mounted within the handle assembly 100 between the right side half-section 102a and the left side half-section 102b of the housing 102 by a first pawl pin 332 at a location wherein the first pawl 330 is in substantial operative engagement with the first rack 310. The first pawl pin 332 extends through a slot 334 defined in the first pawl 330. Similarly, the second pawl 340 is pivotably mounted within the handle assembly 100 between the right side half-section 102a and the left side half-section 102b of the housing 102 by a second pawl pin 342 at a location wherein the second pawl 340 is in substantial operative engagement with the second rack 320. The second pawl pin 342 extends through a slot 344 defined in the second pawl 340.

As shown in FIGS. 2A and 2B, the ratchet assembly 300 further includes a first pawl spring 336 configured to vertically bias the first pawl 330 into operative engagement with the first rack 310 and a second pawl spring 346 configured to vertically bias the second pawl 340 into operative engagement with the second rack 320. The first pawl spring 336 includes a distal hook 336a and a proximal hook 336b configured to latch onto a first set of support pins 116a, 116b of housing 102, respectively, and the second pawl spring 346 includes a distal hook 346a and a proximal hook 346b configured to latch onto a second set of support pins 118a, 118b of housing 102, respectively. It is contemplated that the first and second pawl springs 336, 346 are positioned in a manner configured to maintain a first pawl tooth 338 of the first pawl 330 and a second pawl tooth 348 of the second pawl 340 in registration or engagement with the plurality of first and second rack teeth 312, 322, respectively, as well as to maintain the first and second pawls 330, 340 in a rotated or canted position.

With continued reference to FIGS. 2A and 2B, ratchet assembly 300 further includes release switch 350 at least partially supported within housing 102 of handle assembly 100. In embodiments, release switch 350 includes a slot 350a on a proximal end portion thereof configured for locating a mounting pin 119 of housing 102 such that, the release switch 350 is pivotable thereabout. Release switch 350 is operatively associated with the first pawl 330 and includes an engagement member 350b on a distal end portion thereof that is operable to engage the first pawl 330 to selectively move the first pawl 330, out of operative registration or engagement with the plurality of first rack teeth 312 of the first rack 310, as will be detailed further. In other embodiments, release switch 350 may include various configurations suitable for its intended purpose and may be any ergonomic shape configured to provide access to a user during operation.

In embodiments, the first pawl 330 includes a lockout member 339 extending therefrom and provided to selectively engage the engagement member 350b of release switch 350 to prevent the release switch 350 from moving the first pawl tooth 338 of the first pawl 300 out of registration or engagement with the plurality of first rack teeth 312 of the first rack 310.

Ratchet assembly 300 further includes a release spring 352 supported in housing 102 of handle assembly 100 and operatively associated with the release switch 350 to return the release switch 350 to its un-actuated, home position where the release switch 350 is disengaged from the first pawl 330. It is contemplated that release spring 352 may include any suitable biasing member, such as, for example, a compression spring, a leaf spring, or deforming member extending from release switch 350.

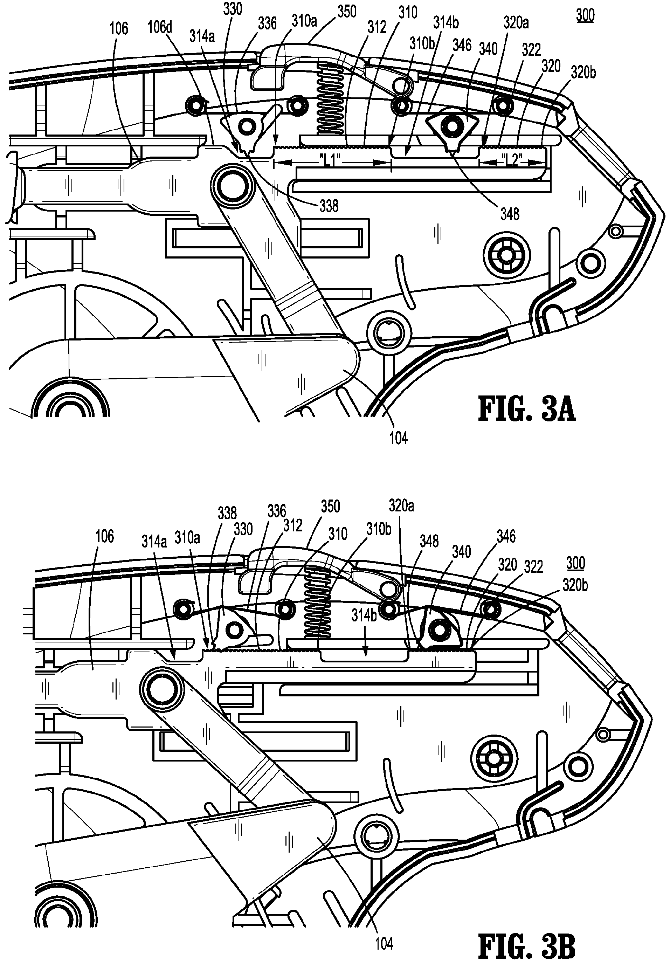

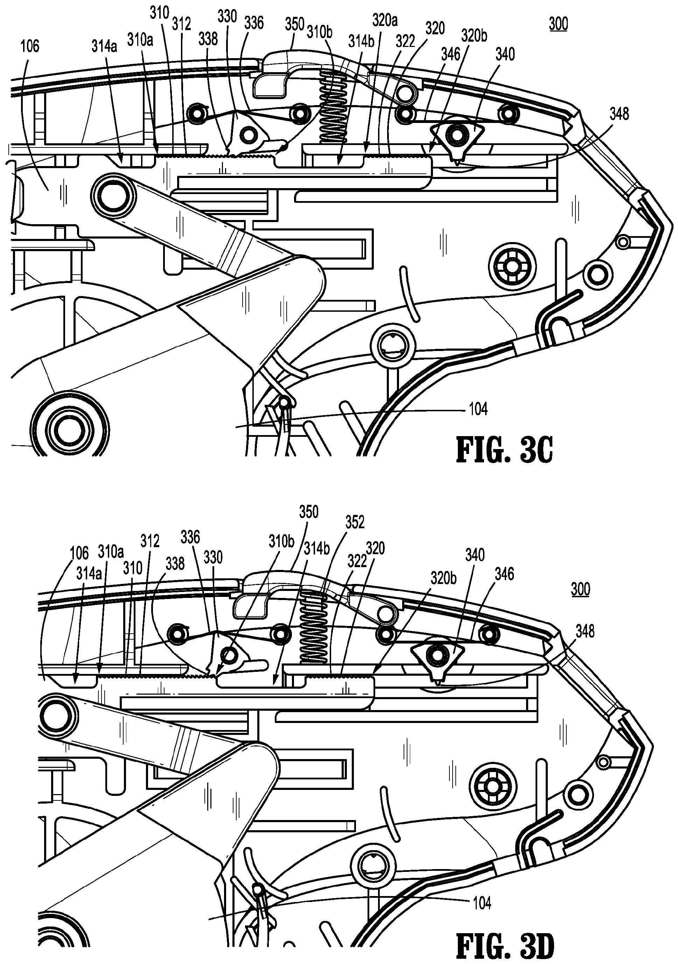

With brief reference to FIG. 3A, in the initial and/or reset position, the first pawl 330 is disposed within distal well 314a and the second pawl 340 is disposed within proximal well 314b.

With continued reference to FIG. 3A and additional reference to FIGS. 3B-3D, a normal actuation of ratchet assembly 300 is disclosed. In use, as trigger 104 is actuated, from a fully un-actuated position, the plurality of first and second rack teeth 312, 322 of the first and second racks 310, 320, respectively, are moved to a first position, into registration or engagement with first pawl tooth 338 and second pawl tooth 348 of the first and second pawls 330, 340, respectively (see FIG. 3B).

First rack 310 has a first length "L1" (see FIG. 3A) which allows the first pawl 330 to reverse over the first rack 310 (from the distal well 314a; see FIG. 3A) as the first rack 310 moves in a distal direction relative to the pair of jaws 250 (see FIG. 1), as trigger 104 reaches a fully actuated position, and advance back over the first rack 310 (from the proximal end 310b of the first rack 310; see FIG. 3D), when the first rack 310 moves in a proximal direction relative to the pair of jaws 250, as trigger 104 reaches a fully un-actuated position. The first length "L1" of first rack 310 defines a full stroke length of trigger 104, drive bar 106 or handle assembly 100 (see FIG. 1), where a clip 290 has been fully formed and fired from surgical clip applier 10.

Second rack 320 has a second length "L2," (see FIG. 3A) which is less than the first length "L1" of first rack 310. The second length "L2" allows the second pawl 340 to reverse over the second rack 320 (from the proximal well 314b; see FIG. 3A) as the second rack 320 moves in the distal direction relative to the pair of jaws 250 (see FIG. 1), as trigger 104 reaches a partially actuated position, and advance back over the second rack 320 (from a position that is proximal of the proximal end 320b of the second rack 320; see FIG. 3D), when the second rack 320 moves in a proximal direction relative to the pair of jaws 250, as trigger 104 reaches a fully un-actuated position. The second length "L2" of second rack 320 defines a partial stroke length of trigger 104, drive bar 106 or handle assembly 100 (see FIG. 1), where a clip 290 has been partially formed, or formed enough to be fired from surgical clip applier 10 and a new clip 290 loaded into the pair of jaws 250 without an inadvertent double loading of clips 290 into the pair of jaws 250. It is contemplated that in the normal actuation of ratchet assembly 300, the first and second pawls 330, 340 and the respective first and second racks 310, 320 cooperate such that the stroke length of trigger 104, drive bar 106 or handle assembly 100 is determined by the greater first length "L1" of first rack 310 to achieve a fully formed clip 290 being fired from surgical clip applier 10.

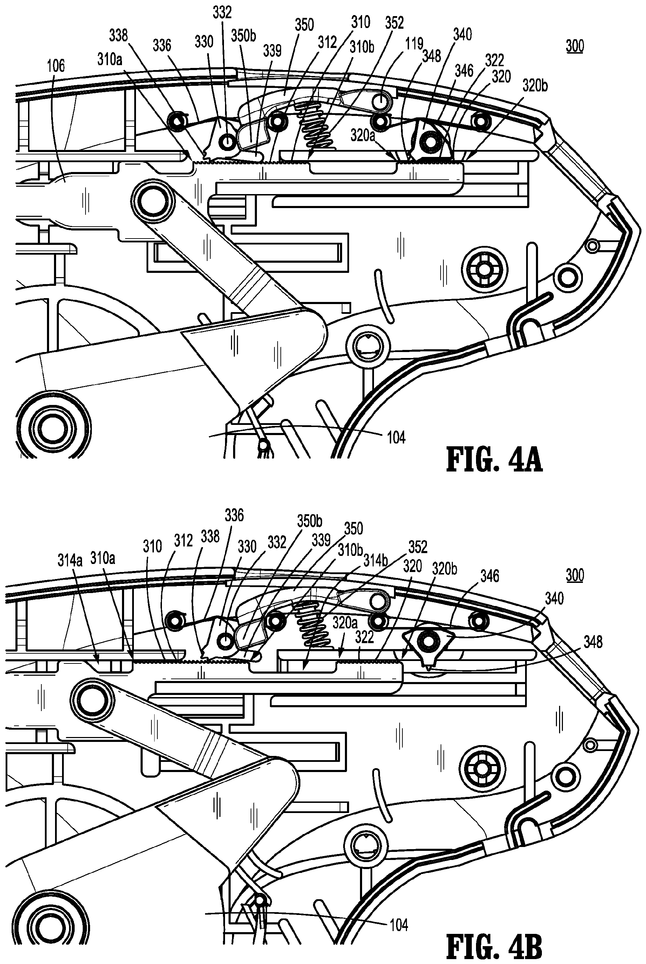

Referring now to FIGS. 4A-4C, a partial actuation of ratchet assembly 300 is disclosed. It is contemplated that a partial actuation of ratchet assembly 300 may enable a user to fire a partially formed clip 290 from surgical clip applier 10 when performing a cholangiogram procedure or the like. It is also contemplated that a partial actuation of ratchet assembly 300 may enable a user to abort a firing of a clip 290 from surgical clip applier 10 if the clip 290 is inadvertently positioned in a wrong location or if a clip 290 is positioned over an obstruction.

In use, with reference to FIG. 4A, after trigger 104 is actuated such that, the plurality of first and second rack teeth 312, 322 of the first and second racks 310, 320, respectively, are moved to the first position, into registration or engagement with first pawl tooth 338 and second pawl tooth 348 of the first and second pawls 330, 340, respectively (see FIG. 3B), release switch 350 is actuated such that release switch 350 pivots about mounting pin 119 of housing 102 and engagement member 350b engages the first pawl 330 to rotate the first pawl 330 about first pawl pin 332. In this manner, the first pawl 330 is moved to a second position, out of registration or engagement with the plurality of first rack teeth 312 of the first rack 310. As shown in FIG. 4A, though release switch 350 is actuated to move the first pawl 330 to the second position, second pawl 340 remains in the first position until the second pawl 340 is moved to a position proximal of the proximal end 320b of the second rack 320 to clear the second rack 320, as shown in FIG. 4B. With first pawl 330 out of registration or engagement with the first rack 310, the stroke length of trigger 104, drive bar 106 or handle assembly 100 is determined by the lesser second length "L2" of second rack 320 (relative to first length "L1" of first rack 310). The engagement between second pawl 340 and second rack 320 for a duration of the lesser second length "L2" prevents an inadvertent return of trigger 104 during a specific portion of the stroke until, a clip 290 loaded into the pair of jaws 250 is partially formed, enough to be fired from surgical clip applier 10, such that a new clip 290 may be loaded into the pair of jaws 250 without an inadvertent double loading of clips 290 into the pair of jaws 250.

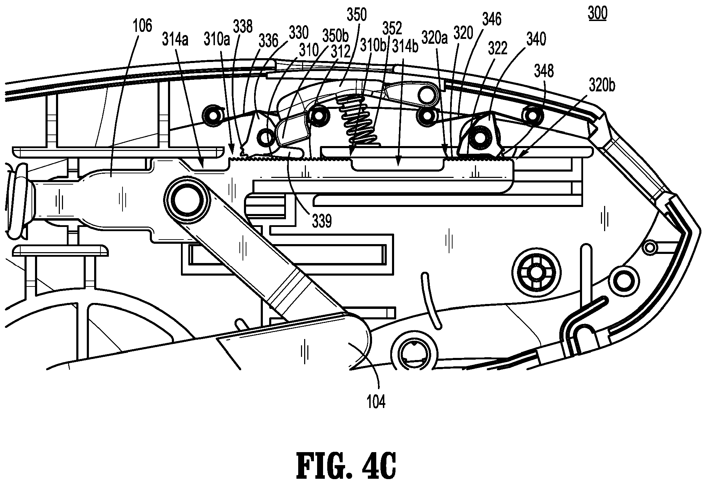

Referring now to FIG. 4C, with release switch 350 still actuated to engage first pawl 330, trigger 104 may be returned to a fully un-actuated position (from its partially actuated position), once second pawl 340 advances back over the second rack 320 and is disposed within the proximal well 314b to complete the partial actuation of ratchet assembly 300. Release switch 350 may then be released such that the release switch 350 is returned to its original position under the bias of release spring 352 and first pawl 330 is disposed within distal well 314a (see FIG. 3A).

In embodiments, it is contemplated that if release switch 350 is released during a portion of the partial actuation of ratchet assembly 300 (for example, see FIG. 4A), such that engagement member 350b of release switch 350 is disengaged from first pawl 330, first pawl spring 336 is provided to return first pawl 330 to the first position, such that the plurality of first rack teeth 312 of the first rack 310 are moved into registration or engagement with first pawl tooth 338 of the first pawl 330. Accordingly, surgical clip applier 10 is returned to the normal actuation of ratchet assembly 300 (see FIGS. 3A-3D), where the stroke length of trigger 104, drive bar 106 or handle assembly 100 is determined by the greater first length "L1" of first rack 310 (relative to second length "L2" of second rack 320) to achieve a fully formed clip 290 being fired from surgical clip applier 10. This feature may be advantageous in instances, such as, for example, where release switch 350 is inadvertently actuated.

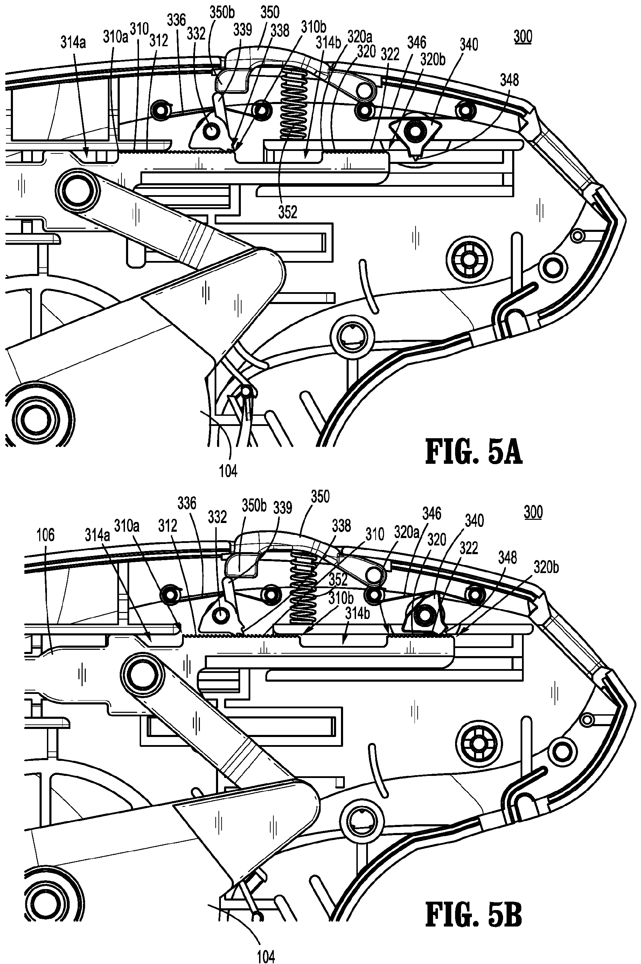

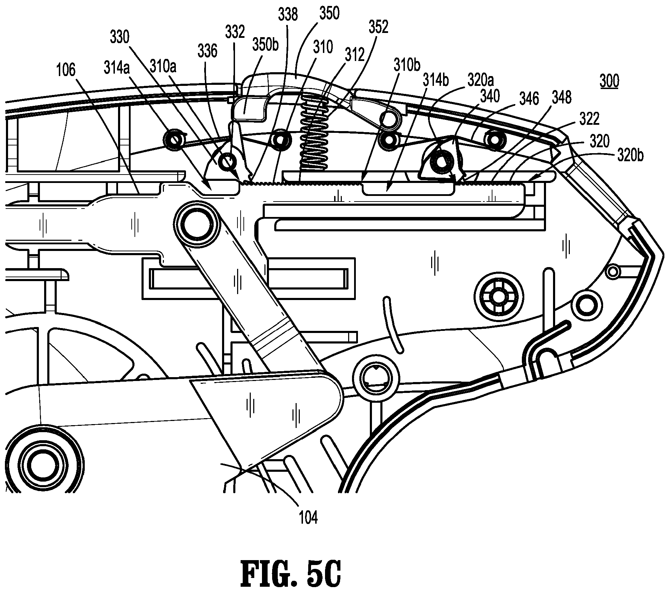

Referring now to FIGS. 5A-5C, a return stroke of the normal actuation of ratchet assembly 300 is disclosed. With trigger 104 in the fully actuated position such that first pawl 330 is disposed at proximal end 310b of first rack 310 and second pawl 340 is disposed at a position proximal of the proximal end 320b of the second rack 320 (see FIG. 5A), trigger 104 is released such that first and second racks 310, 320 are moved in the proximal direction relative to the pair of jaws 250. Concomitantly, first and second pawls 330, 340 are advanced over first and second racks 310, 320, respectively, such that the plurality of first and second rack teeth 312, 322 of the first and second racks 310, 320, respectively, are moved to the first position, into registration or engagement with first pawl tooth 338 and second pawl tooth 348 of the first and second pawls 330, 340, respectively (see FIG. 5B). During the return stroke, first pawl 330 is rotated about first pawl pin 332 such that lockout member 339 of first pawl 330 is positioned to engage the engagement member 350b of release switch 350, if release switch 350 is actuated. In this manner, the registration or engagement between the first pawl tooth 338 of first pawl 330 with the plurality of first rack teeth 312 of the first rack 310 cooperates with lockout member 339 of first pawl 330 to prevent the release switch 350 from engaging the first pawl 330 to rotate or move the first pawl tooth 338 of the first pawl 300 out of registration or engagement with the plurality of first rack teeth 312 of the first rack 310. It is contemplated that such a feature ensures that trigger 104 is moved to the fully un-actuated position, a new clip 290 is loaded into the pair of jaws 250, and ratchet assembly 300 is moved to the initial and/or reset position, where the first pawl 330 is disposed within distal well 314a and the second pawl 340 is disposed within proximal well 314b (see FIG. 3A).

Though the figures of the present disclosure illustrate configurations where the first and second racks 310, 320 are longitudinally aligned on drive bar 106, with release switch 350 being selectively engageable with first pawl 330, it is contemplated that the first and second racks 310, 320 may include configurations where the first and second racks 310, 320 are reversed, stacked, side-by-side, or a combination thereof. Further, it is contemplated that release switch 350 may be selectively engageable with second pawl 340. In addition, it is contemplated that actuating release switch 350 may emit audible and/or tactile feedback to the user.

As noted above, and illustrated in FIG. 6, surgical clip applier 10 includes an endoscopic assembly 200 having hub assembly 210, shaft assembly 220, and the pair of jaws 250. Hub assembly 210 is rotatably mounted on nose 102c (see FIG. 2A) of housing 102 of handle assembly 100 and is connected to a proximal end portion of shaft assembly 220 to provide a three hundred sixty degree rotation of the shaft assembly 220 and the pair of jaws 250 thereon relative to a longitudinal center axis of shaft assembly 220. Hub assembly 210 has a suitable configuration so as to be rotated simply using a clinician's finger.

Endoscopic assembly 200 includes a spindle link 260 for operatively connecting drive bar 106 to a driving mechanism 400 to move the pair of jaws 250 between the spaced-apart configuration and the approximated configuration upon actuation of trigger 104. Specifically, hook member 114 (see FIG. 2B) of drive bar 106 is coupled to a first end 260a of spindle link 260 and a spindle 270 of drive mechanism 400 is coupled to a second end 260b of spindle link 260. In this manner, translation of drive bar 106 in a distal and proximal direction can thus advance and retract spindle 270, respectively.

Drive mechanism 400 further includes an elongated clip channel member 280 for retaining a number of surgical clips 290 shown in an aligned manner above the clip channel member 280. A clip follower 282 and a clip follower spring 284 are provided to urge the surgical clips 290 distally through the elongated clip channel member 280. A channel cover 286 is provided to overlay the elongated clip channel member 280 and retain and guide the clip follower 282 and clip follower spring 284 and the surgical clips 290 distally in the elongated clip channel member 280.

Drive mechanism 400 also has a feed bar 410 for feeding the surgical clips 290 between the pair of jaws 250. Drive mechanism 400 also includes a filler component 420 and a wedge plate 430.

For a more detailed description of the construction and operation of endoscopic assembly 200, reference may be made to U.S. Patent No. 7,637,91 7

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure.

## Claims

1. An endoscopic surgical clip applier, comprising:
an endoscopic assembly (200) including:
a shaft assembly (220); and
a pair of jaw members (250) operatively coupled to, and extending from the shaft assembly; and
a handle assembly (100) including:
a housing (102) selectively connectable to the endoscopic assembly;
a fixed handle (102d) extending from the housing;
a trigger (104) pivotally connected to the fixed handle;
a drive bar (106) disposed within the housing of the handle assembly and operatively coupled to the trigger and the pair of jaw members to move the pair of jaw members between a spaced apart configuration and an approximated configuration upon actuation of the trigger;
a ratchet assembly (300) disposed within the housing of the handle assembly, the ratchet assembly including:
a first rack (310) operatively coupled to the drive bar, the first rack defining a plurality of first rack teeth (312) and having a distal end (310a) and a proximal end (310b), wherein the first rack includes a first length between the distal end and the proximal end thereof; and
a second rack (320) operatively coupled to the drive bar, spaced apart from the first rack, the second rack defining a plurality of second rack teeth (322) and having a distal end (320a) and a proximal end (320b), wherein the second rack includes a second length between the distal end and the proximal end thereof, the second length of the second rack being less than the first length of the first rack,
wherein the ratchet assembly further includes a first pawl (330) mounted within the housing of the handle assembly, the first pawl being selectively engagable with the plurality of first rack teeth of the first rack in a first position thereof; wherein the ratchet assembly further includes a second pawl (340) mounted within the housing of the handle assembly, the second pawl being selectively engageable with the plurality of second rack teeth of the second rack in a first position thereof;
**characterized in that**
the ratchet assembly further includes a release switch (350) at least partially supported within the housing of the handle assembly and operatively associated with the first pawl, the release switch selectively actuatable to move the first pawl from the first position, wherein the first pawl is in registration with the plurality of first rack teeth of the first rack to a second position, wherein the first pawl is out of registration with the plurality of first rack teeth of the first rack.

2. The endoscopic surgical clip applier according to claim 1, wherein the first and second racks are longitudinally aligned on the drive bar.

3. The endoscopic surgical clip applier according to claim 2, wherein the ratchet assembly further includes a distal well (314a) disposed adjacent the distal end of the first rack, wherein the first pawl is located in the distal well in an un-actuated position of the trigger.

4. The endoscopic surgical clip applier according to claim 3, wherein the ratchet assembly further includes a proximal well (314b) disposed between the proximal end of the first rack and the distal end of the second rack, wherein the second pawl is located in the proximal well in the un-actuated position of the trigger.

5. The endoscopic surgical clip applier according to claim 3 or 4, wherein the first rack is disposed in a position distal of the second rack.

6. The endoscopic surgical clip applier according to claim 1, wherein when the release switch is actuated, the second pawl maintains registration with the plurality of second rack teeth of the second rack, in the first position thereof, until the second pawl is disposed in the proximal well or until the second pawl is disposed proximally beyond the proximal end of the second rack; preferably, wherein the drive bar is longitudinally movable upon actuation of the trigger, wherein as the drive bar is moved longitudinally in a first direction, and the release switch is not actuated, the first pawl and the second pawl are moved over the plurality of first rack teeth and the plurality of second rack teeth of the first and the second racks, respectively, such that longitudinal movement of the drive bar in a second, opposite, direction is prevented until the first pawl is disposed in the distal well and the second pawl is disposed in the proximal well or until the first pawl is disposed at the proximal end of the first rack and the second pawl is disposed proximally beyond the proximal end of the second rack.

7. The endoscopic surgical clip applier according to claim 6, wherein the drive bar is longitudinally movable upon actuation of the trigger, wherein as the drive bar is moved longitudinally in a first direction, and the release switch is actuated to move the first pawl out of registration with the plurality of first rack teeth of the first rack, longitudinal movement of the drive bar in a second, opposite, direction is prevented until the second pawl is disposed in the proximal well or until the second pawl is disposed proximally beyond the proximal end of the second rack; preferably wherein as the drive bar is moved longitudinally in the first direction, and the release switch is actuated to move the first pawl out of registration with the plurality of first rack teeth of the first rack, the second pawl is disposed beyond the proximal end of the second rack as the trigger reaches a partially actuated position, wherein the drive bar is longitudinally movable in the second, opposite, direction, as the trigger reaches a fully un-actuated position from the partially actuated position.

8. The endoscopic surgical clip applier according to claim 7, wherein the endoscopic assembly further comprises a plurality of surgical clips (290) slidably disposed within the shaft assembly and selectively formable between the pair of jaw members, wherein when the first pawl is in the first position, the first pawl is in registration with the first rack disposed on the drive bar such that upon actuation of the trigger, the trigger is prevented from reversing the direction of movement thereof until the trigger is moved to a fully actuated position and a distal most surgical clip of the plurality of surgical clips is fully formed between the pair of jaw members.

9. The endoscopic surgical clip applier according to claim 8, wherein when the first pawl housing is in the second position, the first pawl is out of registration with the first rack disposed on the drive bar such that when the second pawl is disposed beyond the proximal end of the second rack and the trigger is moved to the partially actuated position, the trigger is capable of reversing the direction of movement thereof such that the distal most surgical clip of the plurality of surgical clips is partially formed between the pair of jaw members.

10. The endoscopic surgical clip applier according to claim 3, wherein the ratchet assembly further includes a first pawl spring (336) and a second pawl spring (346) supported within the housing of the handle assembly, the first pawl spring being configured to bias the first pawl into engagement with the plurality of first rack teeth of the first rack and the second pawl spring being configured to bias the second pawl into engagement with the plurality of second rack teeth of the second rack.

11. The endoscopic surgical clip applier according to claim 9, wherein the first pawl includes a lockout member (339) extending therefrom, the lockout member provided to selectively engage the release switch, as the trigger moves to the fully un-actuated position, to prevent the release switch from moving the first pawl out of registration with the plurality of first rack teeth of the first rack, to the second position thereof.

12. The endoscopic surgical clip applier according to claim 1, wherein the ratchet assembly further includes a release spring (352) supported in the handle assembly, the release spring operatively associated with the release switch and biased to return the release switch to a home position such that the release switch is disengaged from the first pawl.

13. The endoscopic surgical clip applier, according to claim 1:
the first rack defined on a top portion of the drive bar;
the second rack defined on the top portion of the drive bar;
the first pawl movably mounted within the handle assembly; and
the second pawl movably mounted within the handle assembly,
wherein upon movement of the trigger, a reversal of a direction of movement of the trigger is prohibited until the second pawl is disposed distally beyond the distal end of the second rack or proximally beyond the proximal end of the second rack.

14. The endoscopic surgical clip applier according to claim 13, wherein when the release switch is disengaged from the first pawl such that the first pawl remains in the first position, upon movement of the trigger, the reversal of the direction of movement of the trigger is prohibited until the first pawl is disposed distally beyond the distal end of the first rack or disposed at the proximal end of the first rack.

## Patentansprüche

1. Ein endoskopischer chirurgischer Clip-Applikator, umfassend:
eine endoskopische Anordnung (200), enthaltend:
eine Wellenanordnung (220); und
ein Paar Backenelemente (250), die im Betrieb mit der Wellenanordnung gekoppelt sind und sich von dieser aus erstrecken; und
eine Griffanordnung (100) einschließlich:
eines Gehäuses (102), das selektiv mit der endoskopischen Anordnung verbunden werden kann;
eines festen Griffs (102d), der sich vom Gehäuse aus erstreckt;
eines Auslösers (104), der schwenkbar mit dem festen Griff verbunden ist;
einer Antriebsstange (106), die in dem Gehäuse der Griffanordnung angeordnet und im Betrieb mit dem Auslöser und dem Paar Backenelementen gekoppelt ist, um das Paar Backenelemente bei Betätigung des Auslösers zwischen einer beabstandeten Konfiguration und einer angenäherten Konfiguration zu bewegen;
einer Ratschenanordnung (300), die in dem Gehäuse der Griffanordnung angeordnet ist, wobei die Ratschenanordnung enthält:
eine erste Zahnstange (310), die im Betrieb mit der Antriebsstange gekoppelt ist, wobei die erste Zahnstange mehrere erste Zahnstangenzähne (312) definiert und ein distales Ende (310a) und ein proximales Ende (310b) enthält, wobei die erste Zahnstange eine erste Länge zwischen dem distalen Ende und dem proximalen Ende davon enthält; und
eine zweite Zahnstange (320), die im Betrieb mit der Antriebsstange gekoppelt und von der ersten Zahnstange beabstandet ist, wobei die zweite Zahnstange mehrere zweite Zahnstangenzähne (322) definiert und ein distales Ende (320a) und ein proximales Ende (320b) aufweist, wobei die zweite Zahnstange eine zweite Länge zwischen ihrem distalen Ende und ihrem proximalen Ende enthält, wobei die zweite Länge der zweiten Zahnstange kleiner als die erste Länge der ersten Zahnstange ist,
wobei die Ratschenanordnung ferner eine erste Sperrklinke (330) enthält, die innerhalb des Gehäuses der Griffanordnung montiert ist, wobei die erste Sperrklinke selektiv mit den mehreren ersten Zahnstangenzähnen der ersten Zahnstange in einer ersten Position derselben in Eingriff bringbar ist; wobei die Ratschenanordnung ferner eine zweite Sperrklinke (340) enthält, die innerhalb des Gehäuses der Griffanordnung montiert ist, wobei die zweite Sperrklinke selektiv mit den mehreren zweiten Zahnstangenzähnen der zweiten Zahnstange in einer ersten Position derselben in Eingriff bringbar ist;
**dadurch gekennzeichnet, dass**
die Ratschenanordnung ferner einen Freigabeschalter (350) umfasst, der zumindest teilweise innerhalb des Gehäuses der Griffanordnung gehalten wird und im Betrieb mit der ersten Sperrklinke verbunden ist, wobei der Freigabeschalter selektiv betätigbar ist, um die erste Sperrklinke aus der ersten Position, in der die erste Sperrklinke mit den mehreren ersten Zahnstangenzähnen der ersten Zahnstange ausgerichtet ist, in eine zweite Position zu bewegen, in der die erste Sperrklinke nicht mit den mehreren ersten Zahnstangenzähnen der ersten Zahnstange ausgerichtet ist.

2. Endoskopischer chirurgischer Clip-Applikator nach Anspruch 1, wobei die erste und zweite Zahnstange in Längsrichtung auf der Antriebsstange ausgerichtet sind.

3. Endoskopischer chirurgischer Clip-Applikator nach Anspruch 2, wobei die Ratschenanordnung ferner eine distale Vertiefung (314a) enthält, die angrenzend an das distale Ende der ersten Zahnstange angeordnet ist, wobei sich die erste Sperrklinke in der distalen Vertiefung in einer unbetätigten Position des Auslösers befindet.

4. Endoskopischer chirurgischer Clip-Applikator nach Anspruch 3, wobei die Ratschenanordnung ferner eine proximale Vertiefung (314b) enthält, die zwischen dem proximalen Ende der ersten Zahnstange und dem distalen Ende der zweiten Zahnstange angeordnet ist, wobei sich die zweite Sperrklinke in der proximalen Vertiefung in der unbetätigten Position des Auslösers befindet.

5. Endoskopischer chirurgischer Clip-Applikator nach Anspruch 3 oder 4, wobei die erste Zahnstange in einer Position distal zur zweiten Zahnstange angeordnet ist.

6. Endoskopischer chirurgischer Clip-Applikator nach Anspruch 1, wobei bei Betätigung des Freigabeschalters die zweite Sperrklinke in ihrer ersten Position die Ausrichtung mit den mehreren Zähnen der zweiten Zahnstange beibehält, bis die zweite Sperrklinke in der proximalen Vertiefung angeordnet ist oder bis die zweite Sperrklinke proximal über das proximale Ende der zweiten Zahnstange hinaus angeordnet ist; vorzugsweise, wobei die Antriebsstange bei Betätigung des Auslösers in Längsrichtung beweglich ist, wobei, wenn die Antriebsstange in Längsrichtung in einer ersten Richtung bewegt wird und der Freigabeschalter nicht betätigt wird, die erste Sperrklinke und die zweite Sperrklinke über die mehreren ersten Zahnstangenzähne und die mehreren zweiten Zahnstangenzähne der ersten bzw. der zweiten Zahnstange bewegt werden, so dass eine Längsbewegung der Antriebsstange in einer zweiten, entgegengesetzten Richtung verhindert wird, bis die erste Sperrklinke in der distalen Vertiefung und die zweite Sperrklinke in der proximalen Vertiefung angeordnet ist oder bis die erste Sperrklinke am proximalen Ende der ersten Zahnstange und die zweite Sperrklinke proximal über das proximale Ende der zweiten Zahnstange hinaus angeordnet ist.

7. Endoskopischer chirurgischer Clip-Applikator nach Anspruch 6, wobei die Antriebsstange bei Betätigung des Auslösers in Längsrichtung beweglich ist, wobei, wenn die Antriebsstange in Längsrichtung in einer ersten Richtung bewegt wird und der Freigabeschalter betätigt wird, um die erste Sperrklinke aus der Ausrichtung mit den mehreren ersten Zahnstangenzähnen der ersten Zahnstange zu bewegen, eine Längsbewegung der Antriebsstange in einer zweiten, entgegengesetzten Richtung verhindert wird, bis die zweite Sperrklinke in der proximalen Vertiefung angeordnet ist oder bis die zweite Sperrklinke proximal über das proximale Ende der zweiten Zahnstange hinaus angeordnet ist; wobei vorzugsweise, wenn die Antriebsstange in Längsrichtung in die erste Richtung bewegt wird und der Freigabeschalter betätigt wird, um die erste Sperrklinke aus der Ausrichtung mit den mehreren ersten Zahnstangenzähnen der ersten Zahnstange zu bewegen, die zweite Sperrklinke über das proximale Ende der zweiten Zahnstange hinaus angeordnet wird, wenn der Auslöser eine teilweise betätigte Position erreicht, wobei die Antriebsstange in Längsrichtung in die zweite, entgegengesetzte Richtung bewegbar ist, wenn der Auslöser eine vollständig unbetätigte Position aus der teilweise betätigten Position erreicht.

8. Endoskopischer chirurgischer Clip-Applikator nach Anspruch 7, wobei die endoskopische Anordnung ferner mehrere chirurgische Clips (290) umfasst, die verschiebbar innerhalb der Wellenanordnung angeordnet und selektiv zwischen dem Paar Backenelemente ausgebildet werden können, wobei, wenn sich die erste Sperrklinke in der ersten Position befindet, die erste Sperrklinke mit der ersten Zahnstange ausgerichtet ist, die auf der Antriebsstange so angeordnet ist, dass bei Betätigung des Auslösers eine Umkehrung der Bewegungsrichtung des Auslösers verhindert wird, bis der Auslöser in eine vollständig betätigte Position bewegt wird und ein distalster chirurgischer Clip der mehreren chirurgischen Clips vollständig zwischen dem Paar Backenelemente ausgebildet ist.

9. Endoskopischer chirurgischer Clip-Applikator nach Anspruch 8, wobei, wenn sich das Gehäuse der ersten Sperrklinke in der zweiten Position befindet, die erste Sperrklinke nicht mit der ersten Zahnstange ausgerichtet ist, die auf der Antriebsstange angeordnet ist, so dass, wenn die zweite Sperrklinke jenseits des proximalen Endes der zweiten Zahnstange angeordnet ist und der Auslöser in die teilweise betätigte Position bewegt wird, der Auslöser in der Lage ist, deren Bewegungsrichtung umzukehren, so dass der distalste chirurgische Clip der mehreren chirurgischen Clips teilweise zwischen dem Paar Backenelemente ausgebildet wird.

10. Endoskopischer chirurgischer Clip-Applikator nach Anspruch 3, wobei die Ratschenanordnung ferner eine erste Sperrklinkenfeder (336) und eine zweite Sperrklinkenfeder (346) umfasst, die in dem Gehäuse der Griffanordnung gehalten werden, wobei die erste Sperrklinkenfeder konfiguriert ist, um die erste Sperrklinke in Eingriff mit den mehreren ersten Zahnstangenzähnen der ersten Zahnstange vorzuspannen, und die zweite Sperrklinkenfeder konfiguriert ist, um die zweite Sperrklinke in Eingriff mit den mehreren zweiten Zahnstangenzähnen der zweiten Zahnstange vorzuspannen.

11. Endoskopischer chirurgischer Clip-Applikator nach Anspruch 9, wobei die erste Sperrklinke ein Sperrelement (339) enthält, das sich davon erstreckt, wobei das Sperrelement vorgesehen ist, um selektiv mit dem Freigabeschalter in Eingriff zu kommen, wenn sich der Auslöser in die vollständig unbetätigte Position bewegt, um zu verhindern, dass der Freigabeschalter die erste Sperrklinke aus der Ausrichtung mit den mehreren ersten Zahnstangenzähnen der ersten Zahnstange in deren zweite Position bewegt.

12. Endoskopischer chirurgischer Clip-Applikator nach Anspruch 1, wobei die Ratschenanordnung ferner eine Freigabefeder (352) umfasst, die in der Griffanordnung gehalten wird, wobei die Freigabefeder im Betrieb mit dem Freigabeschalter verbunden und so vorgespannt ist, dass sie den Freigabeschalter in eine Ausgangsposition zurückführt, so dass der Freigabeschalter von der ersten Sperrklinke gelöst wird.

13. Endoskopischer chirurgischer Clip-Applikator nach Anspruch 1, wobei:
die erste Zahnstange auf einem oberen Abschnitt der Antriebsstange definiert ist;
die zweite Zahnstange auf dem oberen Abschnitt der Antriebsstange definiert ist;
die erste Sperrklinke beweglich innerhalb der Griffanordnung montiert ist; und
die zweite Sperrklinke beweglich innerhalb der Griffanordnung montiert ist;
wobei bei Bewegung des Auslösers eine Umkehrung der Bewegungsrichtung des Auslösers verhindert wird, bis die zweite Sperrklinke distal über das distale Ende der zweiten Zahnstange oder proximal über das proximale Ende der zweiten Zahnstange hinaus angeordnet ist.

14. Endoskopischer chirurgischer Clip-Applikator nach Anspruch 13, wobei, wenn der Freigabeschalter von der ersten Sperrklinke gelöst wird, so dass die erste Sperrklinke bei Bewegung des Auslösers in der ersten Position bleibt, die Umkehrung der Bewegungsrichtung des Auslösers verhindert wird, bis die erste Sperrklinke distal über das distale Ende der ersten Zahnstange hinaus oder am proximalen Ende der ersten Zahnstange angeordnet ist.

## Revendications

1. Applicateur endoscopique d'agrafes chirurgicales, comprenant :
un ensemble endoscopique (200) comportant :
un ensemble arbre (220) ; et
une paire d'éléments de mâchoire (250) accouplés de manière fonctionnelle à l'ensemble arbre et s'étendant à partir de celui-ci ; et
un ensemble poignée (100) comportant :
un boîtier (102) pouvant être relié de manière sélective à l'ensemble endoscopique ;
une poignée fixe (102d) s'étendant à partir du boîtier ;
une détente (104) reliée de manière pivotante à la poignée fixe ;
une barre d'entraînement (106) disposée à l'intérieur du boîtier de l'ensemble poignée et accouplée de manière fonctionnelle à la détente et à la paire d'éléments de mâchoire pour déplacer la paire d'éléments de mâchoire entre une configuration espacée et une configuration approximative lors de l'actionnement de la détente ;
un ensemble d'encliquetage (300) disposé à l'intérieur du boîtier de l'ensemble poignée, l'ensemble d'encliquetage comportant :
une première crémaillère (310) étant accouplée de manière fonctionnelle à la barre d'entraînement, la première crémaillère définissant une pluralité de premières dents de crémaillère (312) et ayant une extrémité distale (310a) et une extrémité proximale (310b), la première crémaillère comportant une première longueur entre l'extrémité distale et l'extrémité proximale de celle-ci ; et
une seconde crémaillère (320) étant accouplée de manière fonctionnelle à la barre d'entraînement, espacée de la première crémaillère, la seconde crémaillère définissant une pluralité de secondes dents de crémaillère (322) et ayant une extrémité distale (320a) et une extrémité proximale (320b), la seconde crémaillère comportant une seconde longueur entre l'extrémité distale et l'extrémité proximale de celle-ci, la seconde longueur de la seconde crémaillère étant inférieure à la première longueur de la première crémaillère,
l'ensemble d'encliquetage comportant en outre un premier cliquet (330) monté à l'intérieur du boîtier de l'ensemble poignée, le premier cliquet pouvant être mis en prise de manière sélective avec la pluralité de premières dents de crémaillère de la première crémaillère dans une première position de celle-ci ; l'ensemble d'encliquetage comportant en outre un second cliquet (340) monté à l'intérieur du boîtier de l'ensemble poignée, le second cliquet pouvant être mis en prise de manière sélective avec la pluralité de secondes dents de crémaillère de la seconde crémaillère dans une première position de celle-ci ;
**caractérisé en ce que**
l'ensemble d'encliquetage comporte en outre un allumeur à relâchement (350) au moins partiellement supporté à l'intérieur du boîtier de l'ensemble poignée et associé de manière fonctionnelle au premier cliquet, l'allumeur à relâchement pouvant être actionné de manière sélective pour déplacer le premier cliquet de la première position, le premier cliquet étant en alignement avec la pluralité de premières dents de crémaillère de la première crémaillère dans une seconde position, le premier cliquet étant hors de l'alignement avec la pluralité de premières dents de crémaillère de la première crémaillère.

2. Applicateur endoscopique d'agrafes chirurgicales selon la revendication 1, dans lequel les première et seconde crémaillères sont alignées longitudinalement sur la barre d'entraînement.

3. Applicateur endoscopique d'agrafes chirurgicales selon la revendication 2, dans lequel l'ensemble d'encliquetage comporte en outre un puits distal (314a) disposé adjacent à l'extrémité distale de la première crémaillère, le premier cliquet étant situé dans le puits distal dans une position non actionnée de la détente.

4. Applicateur endoscopique d'agrafes chirurgicales selon la revendication 3, dans lequel l'ensemble d'encliquetage comporte en outre un puits proximal (314b) disposé entre l'extrémité proximale de la première crémaillère et l'extrémité distale de la seconde crémaillère, le second cliquet étant situé dans le puits proximal dans la position non actionnée de la détente.

5. Applicateur endoscopique d'agrafes chirurgicales selon la revendication 3 ou 4, dans lequel la première crémaillère est disposée dans une position distale de la seconde crémaillère.

6. Applicateur endoscopique d'agrafes chirurgicales selon la revendication 1, dans lequel, lorsque l'allumeur à relâchement est actionné, le second cliquet maintient l'alignement avec la pluralité de secondes dents de crémaillère de la seconde crémaillère, dans la première position de celui-ci, jusqu'à ce que le second cliquet soit disposé dans le puits proximal ou jusqu'à ce que le second cliquet soit disposé de manière proximale au-delà de l'extrémité proximale de la seconde crémaillère ; la barre d'entraînement étant de préférence déplaçable longitudinalement lors de l'actionnement de la détente, dans lequel lorsque la barre d'entraînement est déplacée longitudinalement dans une première direction, et que l'allumeur à relâchement n'est pas actionné, le premier cliquet et le second cliquet sont déplacés sur la pluralité de premières dents de crémaillère et la pluralité de secondes dents de crémaillère des première et seconde crémaillères, respectivement, de sorte que le déplacement longitudinal de la barre d'entraînement dans une seconde direction opposée est empêché jusqu'à ce que le premier cliquet soit disposé dans le puits distal et le second cliquet soit disposé dans le puits proximal ou jusqu'à ce que le premier cliquet soit disposé au niveau de l'extrémité proximale de la première crémaillère et le second cliquet soit disposé de manière proximale au-delà de l'extrémité proximale de la seconde crémaillère.

7. Applicateur endoscopique d'agrafes chirurgicales selon la revendication 6, dans lequel la barre d'entraînement est déplaçable longitudinalement lors de l'actionnement de la détente, dans lequel lorsque la barre d'entraînement est déplacée longitudinalement dans une première direction, et l'allumeur à relâchement est actionné pour déplacer le premier cliquet hors de l'alignement avec la pluralité de premières dents de crémaillère de la première crémaillère, le déplacement longitudinal de la barre d'entraînement dans une seconde direction opposée étant empêché jusqu'à ce que le second cliquet soit disposé dans le puits proximal ou jusqu'à ce que le second cliquet soit disposé de manière proximale au-delà de l'extrémité proximale de la seconde crémaillère ; de préférence, dans lequel lorsque la barre d'entraînement est déplacée longitudinalement dans la première direction, et que l'allumeur à relâchement est actionné pour déplacer le premier cliquet hors de l'alignement avec la pluralité de premières dents de crémaillère de la première crémaillère, le second cliquet est disposé au-delà de l'extrémité proximale de la seconde crémaillère lorsque la détente atteint une position partiellement actionnée, la barre d'entraînement étant déplaçable longitudinalement dans la seconde direction opposée, lorsque la détente atteint une position complètement non actionnée à partir de la position partiellement actionnée.

8. Applicateur endoscopique d'agrafes chirurgicales selon la revendication 7, dans lequel l'ensemble endoscopique comprend en outre une pluralité d'agrafes chirurgicales (290) disposées de manière coulissante à l'intérieur de l'ensemble arbre et pouvant être formées de manière sélective entre la paire d'éléments de mâchoire, dans lequel lorsque le premier cliquet est dans la première position, le premier cliquet est en alignement avec la première crémaillère disposée sur la barre d'entraînement de sorte que lors de l'actionnement de la détente, la détente est empêchée d'inverser le sens de déplacement de celle-ci jusqu'à ce que la détente soit déplacée vers une position complètement actionnée et qu'une agrafe chirurgicale la plus distale de la pluralité d'agrafes chirurgicales soit complètement formée entre la paire d'éléments de mâchoire.

9. Applicateur endoscopique d'agrafes chirurgicales selon la revendication 8, dans lequel, lorsque le premier boîtier de cliquet est dans la seconde position, le premier cliquet est hors de l'alignement avec la première crémaillère disposée sur la barre d'entraînement de sorte que lorsque le second cliquet est disposé au-delà de l'extrémité proximale de la seconde crémaillère et que la détente est déplacée vers la position partiellement actionnée, la détente est capable d'inverser le sens de déplacement de celle-ci de sorte que l'agrafe chirurgicale la plus distale de la pluralité d'agrafes chirurgicales est partiellement formée entre la paire d'éléments de mâchoire.

10. Applicateur endoscopique d'agrafes chirurgicales selon la revendication 3, dans lequel l'ensemble d'encliquetage comporte en outre un premier ressort de cliquet (336) et un second ressort de cliquet (346) supportés à l'intérieur du boîtier de l'ensemble poignée, le premier ressort de cliquet étant conçu pour solliciter le premier cliquet en prise avec la pluralité de premières dents de crémaillère de la première crémaillère et le second ressort de cliquet étant conçu pour solliciter le second cliquet en prise avec la pluralité des secondes dents de crémaillère de la seconde crémaillère.

11. Applicateur endoscopique d'agrafes chirurgicales selon la revendication 9, dans lequel le premier cliquet comporte un élément de verrouillage (339) s'étendant à partir de celui-ci, l'élément de verrouillage étant prévu pour mettre en prise de manière sélective l'allumeur à relâchement, lorsque la détente se déplace vers la position complètement non actionnée, pour empêcher l'allumeur à relâchement à partir du déplacement du premier cliquet hors de l'alignement avec la pluralité de premières dents de crémaillère de la première crémaillère, vers la seconde position de celle-ci.

12. Applicateur endoscopique d'agrafes chirurgicales selon la revendication 1, dans lequel l'ensemble d'encliquetage comporte en outre un ressort de libération (352) supporté dans l'ensemble poignée, le ressort de libération étant associé de manière fonctionnelle à l'allumeur à relâchement et sollicité pour ramener l'allumeur à relâchement à une position de repos de sorte que l'allumeur à relâchement est désengagé du premier cliquet.

13. Applicateur endoscopique d'agrafes chirurgicales selon la revendication 1 :
la première crémaillère étant définie sur une partie supérieure de la barre d'entraînement ;
la seconde crémaillère étant définie sur la partie supérieure de la barre d'entraînement ;
le premier cliquet étant monté de manière mobile à l'intérieur de l'ensemble poignée ; et
le second cliquet étant monté de manière mobile à l'intérieur de l'ensemble de poignée ;
lors du déplacement de la détente, une inversion d'une direction de déplacement de la détente étant interdite jusqu'à ce que le second cliquet soit disposé de manière distale au-delà de l'extrémité distale de la seconde crémaillère ou de manière proximale au-delà de l'extrémité proximale de la seconde crémaillère.

14. Applicateur endoscopique d'agrafes chirurgicales selon la revendication 13, dans lequel, lorsque l'allumeur à relâchement est désengagé du premier cliquet de sorte que le premier cliquet reste dans la première position, lors du déplacement de la détente, l'inversion du sens de déplacement de la détente est interdite jusqu'à ce que le premier cliquet soit disposé de manière distale au-delà de l'extrémité distale de la première crémaillère ou disposé au niveau de l'extrémité proximale de la première crémaillère.
